# EUROPÄISCHE PATENTSCHRIFT

(11) **EP 0 691 110 B1**
(45) Veröffentlichungstag und Bekanntmachung des Hinweises auf die Patenterteilung: **10.12.1997**
(21) Anmeldenummer: 95110270.6
(22) Anmeldetag: 01.07.1995
(51) Int. Cl.: A61F 2/46, A61B 17/15

(54) **Vorrichtung zum Einsetzen einer Knieprothese**
Device for the implantation of a knee prosthesis
Dispositif pour la mise en place d'une prothèse de genou

(30) Priorität: 08.07.1994 DE 4423717
(43) Veröffentlichungstag der Anmeldung: 10.01.1996
(73) Patentinhaber: ESKA Implants GmbH & Co., 23556 Lübeck (DE)
(72) Erfinder: Grundei, Hans, Dr., D-23558 Lübeck (DE)
(74) Vertreter: Fuchs Mehler Weiss

(56) Entgegenhaltungen:
- EP-A- 0 243 109
- EP-A- 0 466 659
- EP-A- 0 474 320
- US-A- 4 487 203

## Beschreibung

Die vorliegende Erfindung betrifft eine Vorrichtung zur Festlegung von Resektionsflächen am Femur und an der Tibia des Menschen zur Vorbereitung einer Implantation einer Kniegelenkstotalendoprothese.

Bevor eine Kniegelenkstotalendoprothese, wie sie beispielsweise bekannt ist aus der DE-39 22 294 C1 und der DE-41 41 757 C1, implantiert werden kann, müssen die angrenzenden Knochenbereiche des Femurs und der Tibia in geeigneter Weise reseziert werden, um Normanlageflächen entsprechend der Vorgabe der Geometrie der Endoprothese zu schaffen. Heute werden üblicherweise die Frontalen der Tibia und des Femurs reseziert. Wenigstens der Femur erhält darüber hinaus einen sogenannten Dorsal- sowie einen Ventralschnitt, da die heute üblichen Femurteile von Totalendoprothesen so ausgebildet sind, um die dadurch entstehenden Resektionsflächen (dorsal, ventral und frontal) nach Art einer Klammer einzufassen.

Die exakte Lage der Resektionslinien dabei zu wählen ist recht schwierig.

Bislang jedenfalls wurde vom Operateur mit einiger Erfahrung bei solchen Operationen die Ausrichtung von Tibia zum Femur mit einer Valgität von 2,5 bis 7,5° durch Inaugenscheinnahme vorgenommen. Entsprechend dieser Einstellung wurden dann die Sägelehren angesetzt und mit einer oszillierenden Knochensäge die Resektionen durchgeführt.

Dies erfordert freilich große Erfahrung des Operateurs. Anfänger haben damit naturgemäß größere Schwierigkeiten, ebenso wie sehr wohl erfahrene Operateure unter Streßsituationen während des Eingriffs.

Grundsätzlich läßt sich sagen, daß sowohl der Ventral- als auch der Dorsalschnitt am Femurknochen so ausgerichtet werden müssen, daß sie parallel zur Frontalresektionsfläche an der Tibia verlaufen müssen, damit die Kniegelenkstotalendoprothese achsgerecht ausgerichtet ist. Richtig ausgerichtet ist die Endoprothese dann, wenn keinerlei einseitige Belastungen in dem Gelenk auftreten, die beispielsweise zu einseitig erhöhtem Verschleiß der Gleitflächen in dem Tibiateil der Endoprothese durch die nachgebildeten Kondylen des Femurteils des Gelenks führen können.

Vor diesem Hintergrund ist es die Aufgabe der vorliegenden Erfindung, eine Vorrichtung anzugeben, die es in einfacher, aber präziser Weise gestattet, die Resektionsflächen am Femur und an der Tibia zur Vorbereitung einer Implantation einer Kniegelenkstotalendoprothese so festzulegen, daß der Ventral- und der Dorsalschnitt am Femur parallel vorgenommen wird zu der frontalen Resektionsfläche in der Tibia.

Gelöst wird diese Aufgabe durch die Vorrichtung mit den Merkmalen des Hauptanspruchs. Weitere vorteilhafte Weiterbildungen ergeben sich aus den Unteransprüchen.

Ausgegangen wird von einer Vorrichtung, die im wesentlichen besteht aus einem lösbar im Bereich der tuberositas tibiae arretierbaren und dort in reproduzierbarer Weise lagegenau positionierbaren Tibiateil und aus einer femoralen Sägelehre.

Erfindungsgemäß ist vorgesehen, daß das Tibiateil im wesentlichen aus zwei parallelen Platten, die mittels einer geeigneten Vorschubeinrichtung zueinander exakt parallel verfahrbar sind, und aus einem Anschlagsblock, der zur Anlage an der Tibia im Bereich der tuberositas tibiae dient, besteht, wobei die eine Platte feststehend exakt senkrecht zur Hauptachse des Anschlagsblockes angeschlagen ist und für die Auflage frontal auf der Tibia vorgesehen ist, daß die femorale Sägelehre andererseits im wesentlichen aus einem Anlageblock besteht, der exakt quaderförmig ausgebildet ist und mit seiner größten Stirnfläche zur frontalen Anlage an dem Femur vorgesehen ist, und zwar so, daß er um seine zentrale Schwenkachse schwenkbar ist, und daß das Tibiateil und die femorale Sägelehre in situ, d. h. während des Eingriffs so zueinander in Lagebeziehung gebracht werden, daß beim Verfahren der bewegbaren Platte des Tibiateils durch Betätigung der Vorschubeinrichtung fort von der feststehenden Platte zwischen ihr und der tibiawärtsweisenden Stirnfläche der Sägelehre gegebenenfalls unter Ausführung einer Schwenkbewegung um die Schwenkachse der Sägelehre zumindest ein linienförmiger Kontakt entsteht.

Operativ wird folgendermaßen vorgegangen: zunächst wird das Tibiateil auf einen als Fädelhilfe dienenden Führungsspieß bekannter Art aufgezogen. Dabei ist vorher die Vorschubeinrichtung so betätigt worden, daß die bewegbare Platte praktisch auf der feststehenden Platte aufliegt. Das Tibiateil wird so weit bewegt, daß der Anschlagsblock an der Tibia im Bereich der tuberositas tibiae zur Anlage kommt und die bewegbare Platte auf der frontalen Stirnseite der Tibia anschlägt. In dieser Stellung wird das Tibiateil an der Tibia arretiert, beispielsweise durch Fixationsnägel, wie weiter unten näher beschrieben wird.

In dieser Lage kann nun der Führungsspieß, der über ein Führungselement mit dem Tibiateil in Verbindung steht, zusammen mit diesem entfernt werden. Die Lage des Tibiateils zur Tibia bleibt davon unberührt.

Die femorale Sägelehre besteht im einfachsten Ausführungsbeispiel aus einem Anlageblock, der exakt quaderförmig ausgebildet ist und der in seinem geometrischen Zentrum eine Durchbohrung aufweist, durch welchen ebenfalls ein Führungsspieß gefädelt werden kann, um in den Markraum des Femurs gestoßen zu werden. Dieser Führungsspieß fixiert den Anlageblock der Sägelehre, läßt jedoch dessen Schwenken um seine Hauptachse zu. Das Tibiateil und die femorale Sägelehre werden nun in eine solche Lagebeziehung versetzt, daß durch Betätigung der Vorschubeinrichtung des Tibiateils die bewegbare Platte von der feststehenden Platte abhebt und sich in Richtung auf die tibiawärtsweisende Stirnfläche des Anlageblockes der femoralen Sägelehre bewegt. An irgendeinem Punkt wird die bewegbare Platte als erstes diese Stirnfläche berühren. Ein weiterer Vorschub wird den Anlageblock in eine leichte Schwenkbewegung versetzen, solange, bis der Kontakt zwischen der Stirnfläche und der bewegbaren Platte zumindest linienförmig, maximal aber flächenhaft hergestellt ist. Letzteres trifft dann zu, wenn die Schwenkachse des Anlageblockes exakt senkrecht auf der Tibiaachse steht. Dies ist freilich nicht immer der Fall und für die achsgerechte Ausführung der Resektion, die durch die erfindungsgemäße Vorrichtung festgelegt wird, auch nicht notwendig.

Der Anlageblock wird nun mit Fixationsnägeln auf der Frontalen des Femurs befestigt und der Führungsspieß aus dem Femurmarkraum entfernt. In dem Anlageblock sind Sägeschlitze vorgesehen, durch welche hindurch ein Blatt einer oszillierenden Knochensäge greifen kann, welche die so festgelegte Resektionsflächen (ventral und dorsal) im Femur erzeugen kann.

Der Kern der erfindungsgemäßen Vorrichtung ist, daß die genauen Winkelbeziehungen der Tibia (Frontale gegenüber Tibiaachse) durch die Vorschubbewegung der bewegbaren Platte und die darauf erfolgende Schwenkung des Anlageblockes der femoralen Sägelehre abgebildet werden auf den Femur.

Mit - soweit ersichtlich - bislang nicht bekannter Präzision werden durch die Parallelverschiebung der bewegbaren Platte zu der feststehenden Platte, welche die Frontale der Tibia repräsentiert, und durch die Ausführung der Schwenkbewegung der femoralen Sägelehre die individuellen Achsverhältnisse bei jedem einzelnen Patienten abgebildet und so der optimale Sitz der zu implantierenden Endoprothese gewährleistet.

Zur bereits erwähnten Arretierung des Anschlagblockes an der Tibia nach erfolgter Ausrichtung mit der Tibiaachse, welche mit dem Führungsspieß durchgeführt werden kann, weist der Anschlagblock des Tibiateils vorzugsweise eine Reihe von Durchbohrungen auf, durch welche hindurch Fixationsnägel gesetzt und in den Tibiaknochen eingeschlagen werden können. Zur lagegenauen Arretierung sind wenigstens zwei Durchbohrungen vonnöten. Damit das Tibiateil entfernt werden kann, jedoch hinterher wieder in der exakten Lage an die Tibia zu bringen ist, weisen die Fixationsnägel keine Köpfe auf, sondern sind im Prinzip eher als Pins gleichbleibenden Durchmessers zu bezeichnen.

Vorzugsweise ist die bewegliche Platte des Tibiateils distal mit zwei Tibiaanschlägen versehen. Diese Tibiaanschläge sind beim Ansatz des Tibiateils an die Tibia quasi als Fühler zu bezeichnen, da mit ihnen nach Frontalanlage an bzw. auf der Tibia die Ebene festgelegt wird, welche mit Betätigung der Vorschubeinrichtung hinterher parallel verschoben wird. Am Rande sei bemerkt, daß nach schlußendlicher Resektion auch der Tibiafrontalen eine Kontrollmöglichkeit mit diesen Tibiaanschlägen gegeben ist dahingehend, ob nämlich der Frontalschnitt auf der Tibia in der korrekten Ebene durchgeführt wurde. Dies ist nämlich nur dann der Fall, wenn das Tibiateil am Schluß der Vorbereitung der Implantation wieder lagegetreu an der Tibia arretiert wird und beide Tibiaanschläge gleichzeitig die frontale Resektionsfläche der Tibia berühren.

Das bereits erwähnte Führungselement, mittels dessen das Tibiateil anfangs mit der Tibiaachse ausgerichtet wird, ist gemäß einer bevorzugten Ausführungsform in einen Führungskasten einsetzbar, welcher von der beweglichen Platte des Tibiateils getragen wird. Das Führungselement verläuft dann von ventral nach dorsal und weist an seinem dorsalen Ende eine Führungsbuchse für den erwähnten Führungsspieß auf, welcher in den Markraum der Tibia gestoßen wird.

Alternativ oder zusätzlich zu der Ausrichthilfe des Tibiateils in Form des erwähnten Führungsspießes kann außen am Anschlagsblock eine externe Ausrichthilfe für das Tibiateil angebracht sein, und zwar in Form eines Peilstabes. Der Operateur richtet diesen Peilstab exakt mit der sichtbaren Achse der Tibia aus. Die weiteren Schritte entsprechen jenen, wie sie weiter oben beschrieben sind.

Die nachfolgend beschriebenen vorteilhaften Weiterbildungen betreffen das zweite Teil der Vorrichtung, nämlich die femorale Sägelehre.

Bevorzugt wird, wenn die Schwenkachse für die femorale Sägelehre durch einen femorale Führungsspieß gebildet ist, welcher in den Markraum des Femurs einsetzbar ist. Ein solcher femoraler Führungsspieß ist bekannt und kann beispielsweise in besonders bevorzugter Weise gemäß der DE-U-93 17 230 ausgebildet sein. Zwecks Einstellung der Valgität kann im übrigen vorgesehen sein, daß die Schwenkachse der femoralen Sägelehre auf der Bohrungsachse einer auf dem Anlageblock ausgebildeten Exzenterbuchse liegt. Je nach Exzentrität der Durchbohrungen der Buchse wird die Valgusstellung (Valgität) eingestellt im Bereich 2,5° und 7,5°.

In dem Anlageblock ist wenigstens ein Schlitz vorgesehen, durch welchen ein Blatt einer oszillierenden Knochensäge greifen kann. Damit wird schließlich die Resektion dorsal und ventral am Femur durchgeführt.

Um die Lage des Anlageblockes am Femur noch genauer zu definieren, ist vorzugsweise vorgesehen, daß von diesem ein Winkelansatz abgeht, der einen femurwärts in einem Winkel von exakt 90° zur ventral weisenden Stirnfläche des Anlageblockes stehenden Schenkel trägt. Dieser Schenkel hält an seinem distalen Ende einen Femurkontaktfühler, der als ein zum Femur weisender und exakt senkrecht auf dem Schenkel stehender Bolzen ausgebildet ist. Die Lage der Sägelehre auf der Frontalen des Femurs wird in diesem Ausführungsbeispiel exakt festgelegt einmal durch den Führungsspieß, auf dem die Sägelehre aufgefädelt ist und der in den Markraum des Femurs gestoßen wird, und zum anderen durch den beschriebenen Winkelansatz, Schenkel und Femurkontaktfühler, die in exakter Winkelbeziehung zu dem Anlageblock stehen.

Auf die Sägelehre ist - ähnlich wie das Tibiateil - lösbar arretierbar mittels durch sie hindurchlaufender Durchbohrungen für die Aufnahme von Fixationsnägeln oder Pins.

In aller Kürze seien hier die wichtigstens Schritte der Anwendung einer Ausführungsform der erfindungsgemäßen Vorrichtung chronologisch aufgeführt:
1. Auffädeln des Tibiateils auf den Tibiaführungsspieß (soweit vorhanden)
2. Eröffnen des Markraumes der Tibia und Hineinstoßen des Führungsspießes, bis das Tibiateil mit aufeinanderruhenden Platten auf der Tibia zu liegen kommt, wobei vorzugsweise die bewegliche Platte mit Tibiaanschlägen versehen sind.
3. Arretierung des Tibiateils mittels Fixationsnägeln
4. Tibiaführungsspieß und das Führungselement werden entfernt.
5. Auflädeln der femoralen Sägelehre auf den femoralen Führungsspieß.
6. Eröffnen des Markraumes des Femurs und Hineinstoßen des Führungsspießes bis zur frontalen Anlage des Anlageblockes am Femur.
7. Durchführung der Parallelverschiebung der beweglichen Platte zur feststehenden Platte des Tibiateils bis zumindest linienförmiger Kontakt zur Sägelehre hergestellt ist.
8. Arretierung der femoralen Sägelehre auf der Frontalen des Femurs in dieser Lage.
9. Das Tibiateil wird (bis auf die Fixationsnägel) entfernt.
10. Die femoralen Resektionsschnitte (dorsal und ventral) werden ausgeführt.
11. Die femoralen Resektionshilfen werden entfernt.
12. Wiederaufsetzen des Tibiateils an die Tibia.
13. Lagegerechte Wiederherstellung durch die im Knochen verbliebenen Fixationsnägel.
14. Durchführung der tibialen Resektionsschnitte.
15. Kontrolle des Tibiaschnitts durch die Tibiaanschläge. Korrekter Schritt liegt vor, wenn beide Anschläge gleichzeitig auf der Resektionsfläche zur Anlage kommen.

Nachfolgend wird die Erfindung anhand eines Ausführungsbeispiels gemäß der Zeichnungsfiguren näher erläutert. Hierbei zeigt:
- Fig. 1: die gesamte Vorrichtung in Lagebeziehung zum Femur und zur Tibia,
- Fig. 2: die Ansicht des Tibiateils, gesehen in Richtung des Pfeiles II in Fig. 1,
- Fig. 3: die Schnittansicht des Tibiateils mit angekoppeltem Führungselement und Führungsspieß, und
- Fig. 4: die schematische Ansicht auf die Tibia und dem Femur in gebeugter Lagebeziehung mit angesetzter Vorrichtung.

Nachfolgend sind die gleichen Teile mit denselben Bezugszeichen versehen.

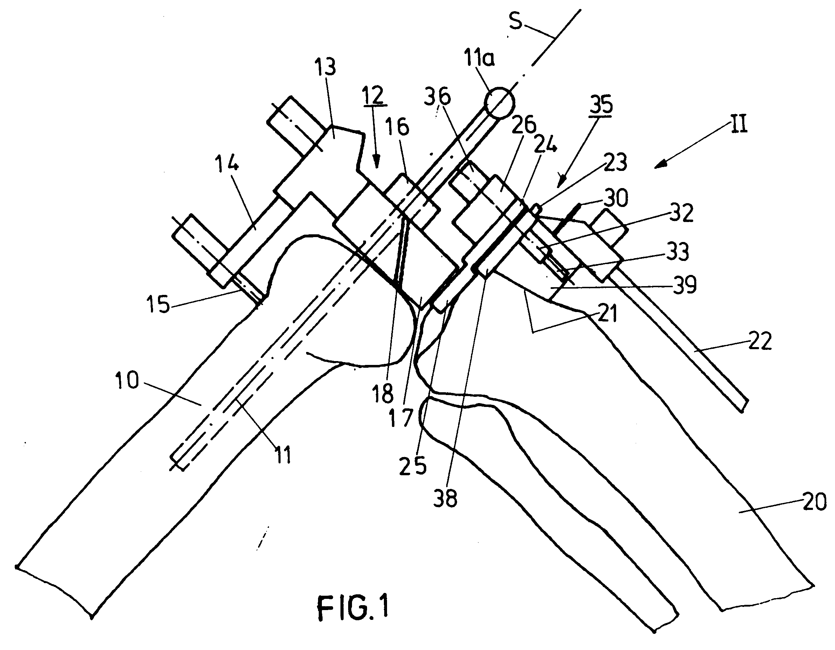

In Fig. 1 ist die gesamte Vorrichtung gemäß einer Ausführungsform abgebildet, und zwar wie sie am Femur 10 bzw. an der Tibia 20 angebracht ist.

Im wesentlichen besteht die Vorrichtung aus der femoralen Sägelehre 12 und dem Tibiateil 35.

Die Funktion der erfindungsgemäßen Vorrichtung soll kurz anhand der Fig. 1 dargelegt werden.

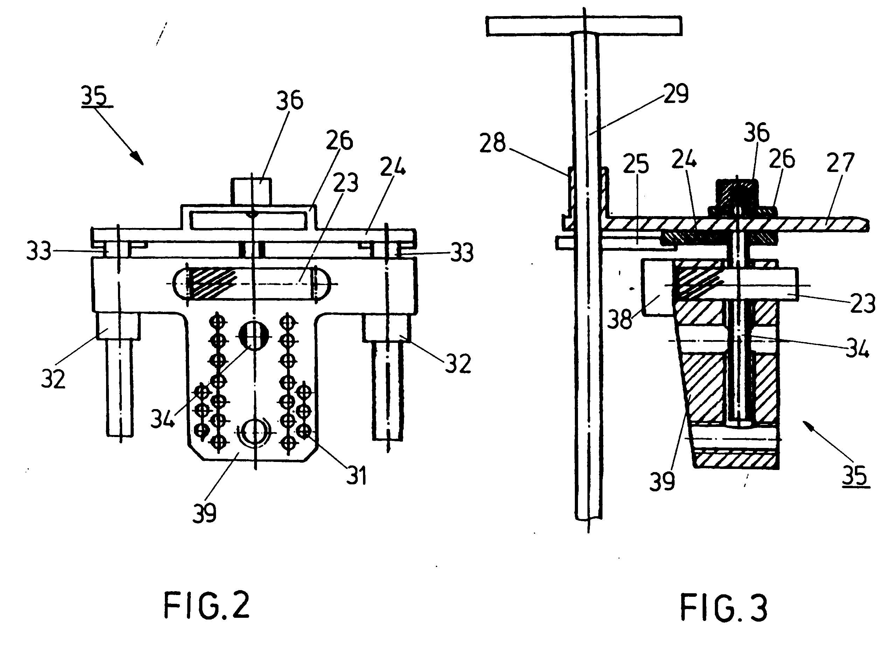

Das Tibiateil 35 liegt an der Tibia an, und zwar mit seinem Anschlagblock 39 im Bereich der tuberositas tibiae 21. Die Ausrichtung ist hierbei erfolgt durch die externe Ausrichthilfe 22, dergestalt, daß diese parallel zur Tibiaachse verläuft. Das Tibiateil 35 ist an der Tibia 20 arretiert mittels Fixationsnägeln 30. Die Ausrichtung des Tibiateils 35 ist so erfolgt, daß die Tibiaanschläge 25 an der beweglichen Platte 24 auf der Frontalen der Tibia 20 aufliegen. Die bewegliche Platte 24 liegt in diesem Stadium auf der feststehenden Platte 38 des Tibiateils 35 auf. Dies wird erreicht durch eine entsprechende Betätigung der Vorschubeinrichtung, die hier aus einer Rändelschraube 23 und der im Inneren des Anschlagsblockes 39 verlaufenden Gewindespindel 34 (Fig. 2, Fig. 3) besteht.

In dieser Ansicht ist noch eine der beiden Führungshülsen 32 für Führungsstangen 33 zu sehen, welche die bewegliche Platte 24 bei der Betätigung der Vorschubeinrichtung 23 exakt parallel zur feststehenden Platte 38 führen.

Erkennbar beim Tibiateil 35 ist darüber hinaus der mit der beweglichen Platte 24 verbundene Führungskasten 26 in Seitenansicht, in den zur Ausrichtung des Tibiateils 35 mittels eines Führungsspießes 29 (Fig. 3) zur Tibiaachse alternativ oder zusätzlich zur Ausrichtung mittels der externen Ausrichthilfe 22 ein Führungselement 27 (Fig. 3) setzbar ist. Dieses Führungselement 27 trägt an seinem dorsalen Ende eine Führungsbuchse 28 für den tibialen Führungsspieß 29.

Die femorale Sägelehre 12 besteht im wesentlichen aus dem Anlageblock 17, welcher frontal auf den Femur 10 aufliegt.

Zwar ist der Anlageblock 17 an der Frontalen des Femurs 10 vorzugsweise mit Fixationsnägel arretierbar, in der dargestellten Lage jedoch ist der Anlageblock 17 aber schwenkbar um die Schwenkachse S gelagert, die im dargestellten Fall gebildet ist durch den Führungsspieß 11 mit Hantel 11a, der in den Markraum des Femurs 10 gestoßen ist. Vorliegend sitzt auf dem Anlageblock 17 eine Exzenterbuchse 16 mit einer exzentrischen Bohrung. Die Exzentrität dieser Bohrung gibt eine Valgität zwischen 2,5 bis 7,5° vor.

Der Anlageblock 17 ist von exakt quaderförmiger Form. Zur noch genaueren Ausrichtung der femoralen Sägelehre 12 weist dieser einen Winkelansatz 13 auf, der in der dargestellten Weise vom Anlageblock 17 absteht. Der Winkelansatz 13 trägt einen femurwärts in einem Winkel von 90° zu der ventral weisenden Stirnfläche des Anlageblockes 17 stehenden Schenkel 14. Dieser weist an seinem distalen Ende einen sogenannten Femurkontaktfühler 15 auf, der hier als exakt senkrecht auf dem Schenkel 14 stehender und hin zum Femur 10 weisender Bolzen ausgebildet ist. Der Femurkontaktfühler liegt auf der ventralen Seite des Femurs 10 auf.

In der abgebildeten Lage wird nun die Rändelschraube 23 so bewegt, daß die bewegliche Platte 24 von der feststehenden Platte 38 abhebt und sich in Richtung auf den Anlageblock 17 bewegt. Der erste Kontakt zwischen dem Tibiateil 35 und der Sägelehre 12 findet punktuell statt zwischen einem Tibiaanschlag 25 und der tibiawärtsweisenden Stirnfläche des Anlageblockes 17. Bei weiterer Betätigung der Rändelschraube 23 schwenkt der Anlageblock 17 so, bis schließlich ein wenigstens linienförmiger Kontakt zwischen den Tibiaanschlägen 25 und dem Anlageblock 17 vorliegt. Mit dieser Parallelverschiebung der Tibiafrontalen auf die Sägelehre wird erreicht, daß der anzubringende Dorsal- und der Ventralschnitt am Femur parallel verläuft zur Frontalen der Tibia.

In der Sägelehre 12 sind Schlitze 18 (nur einer ist dargestellt) für die oszillierende Knochensäge vorgesehen, um das Blatt der Säge bei dem exakten Schnitt zu führen. Hierzu wird die Sägelehre 12 an der Frontalen des Femurs 10 beispielsweise durch Fixationsnägel (nicht dargestellt) arretiert, die durch Durchbohrungen (nicht dargestellt) durch den Anlageblock 17 verlaufen. Danach kann der Führungsspieß 11 durch Ziehen an der Hantel 11a entfernt werden, ebenso wie die Exzenterbuchse 16, so daß der Operateur freien Zugang zu den Schlitzen 18 hat, um die notwendigen Resektionsflächen am Femur 10 herzustellen.

Fig. 2 zeigt die Ansicht auf das Tibiateil 35 in Richtung des Pfeiles II in Fig. 1. Hervorzuheben sind hier die Durchbohrungen 31 für die Fixationsnägel 30. Im dargestellten Ausführungsbeispiel sind eine ganze Reihe von Durchbohrungen 31 vorgesehen, damit eine Arretierung mit großer Wahrscheinlichkeit möglich ist in dem möglicherweise beschädigten Knochen. Wenigstens zwei Durchbohrungen sind allerdings notwendig, um eine Reproduzierbarkeit der Lage des Tibiateils 35 an der Tibia 20 zu erhalten.

Deutlich wird aus Fig. 2 die Parallelführung der beweglichen Platte 24 gegenüber dem übrigen Tibiateil 35. Erreicht wird dies durch Führungshülsen 32 und darin geführten Führungsstangen 33, die von unten an der beweglichen Platte 24 befestigt sind. Die Vorschubbewegung wird durch Drehung der Rändelschraube 23 erzielt, die mit einer im Inneren des Anschlagblockes 39 geführten Gewindespindel 34 zusammenarbeitet.

Klar erkennbar in dieser Ansicht ist auch der schon erwähnte Führungskasten 26 für das Führungselement 27 (Fig. 3). Der Führungskasten 26 trägt vorliegend einen Kugelkasten 36, in dem eine unter einer Federkraft stehende Kugel gelagert ist, derart, daß sie teilweise in den Führungskasten 26 hineinreicht. Diese Kugel arbeitet mit dem Führungselement 27 so zusammen, daß sie die Rückhaltekraft für das Führungselement 27 erhöht, damit das Führungselement 27 in dem Führungskasten 26 nicht ohne weiteres eine einmal eingestellte Position verlassen kann, sondern nur gezielt mit einem gewissen Kraftaufwand verschoben werden kann.

Wie schon erwähnt, weist das Führungselement 27 an seinem dorsalen Ende eine Führungsbuchse 28 auf, in welche der Führungsspieß 29 setzbar ist. Die Längsachse der Führungsbuchse 28 steht dabei exakt senkrecht auf dem Schenkel des Führungselementes 27.

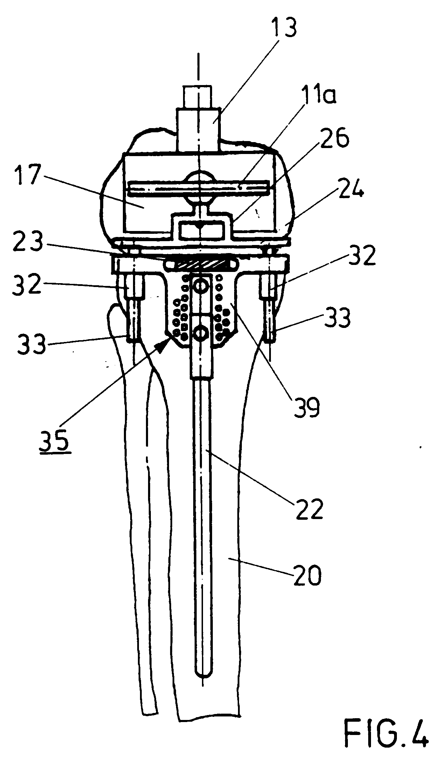

Fig. 4 schließlich soll lediglich zur Abrundung des Gesamtbildes beitragen. Sämtliche Teile sind bereits anhand der vorstehenden Beschreibung und der übrigen Zeichnungsfiguren erläutert worden. Fig. 4 zeigt die Ansicht der gesamten Vorrichtung von ventral gesehen. Erwähnenswert bleibt hier noch die externe Ausrichthilfe 22 in Form eines Peilstabes. Dieser wird vom Operateur mit der Tibiaachse ausgerichtet. Wie ersichtlich, ist das Führungselement 27 (Fig. 3) aus dem Führungskasten 26 und damit auch der Führungsspieß 29 entfernt worden und die Vorrichtung ist bereit für die Parallelverschiebung der frontalen Tibiaebene hin zur femoralen Sägelehre 12.

### Bezugszeichenliste

- 10: Femur
- 11: Führungsspieß für Femurmarkraum
- 11a: Hantel von 11
- 12: femorale Sägelehre
- 13: Winkelansatz 90°
- 14: Schenkel
- 15: Femurkontaktfühler
- 16: Exzenterbuchse zur Vorgabe einer Valgität von 2,5 - 7,5°
- 17: Anlageblock von 12 an Femur (frontal)
- 18: Schlitz in 17 für oszillierende Knochensäge
- 20: Tibia
- 21: tuberositas tibiae
- 22: externe Ausrichthilfe (optional oder alternativ zu Führungsspieß 29)
- 23: Rändelschraube
- 24: Hubplatte
- 25: Tibiaanschlag
- 26: Führungskasten für Führungselement 27
- 27: Führungselement
- 28: Führungsbuchse für Führungsspieß 29
- 29: Führungsspieß für Tibiamarkraum
- 30: Fixationsnägel
- 31: Durchbohrungen für Fixationsnägel
- 32: Führungshülsen für Führungsstangen 33
- 33: Führungsstangen
- 34: Gewindespindel, betätigbar durch Rändelschraube 23
- 35: Tibiateil der Vorrichtung
- 36: Kugelkasten
- 38: feststehende Platte
- 39: Anschlagsblock

## Patentansprüche

1. Vorrichtung zur Festlegung von Resektionsflächen am Femur und an der Tibia zur Vorbereitung einer Implantation einer Kniegelenkstotalendoprothese, bestehend im wesentlichen aus einem lösbar im Bereich der tuberositas tibiae arretierbaren und dort in reproduzierbarer Weise lagegenau positionierbaren Tibiateil (35) und aus einer femoralen Sägelehre (12),
dadurch gekennzeichnet,
daß das Tibiateil (35) im wesentlichen aus zwei parallelen Platten (24, 38), die mittels einer geeigneten Vorschubeinrichtung (23, 34) zueinander exakt parallel verfahrbar sind, und aus einem Anschlagblock (39), der zur Anlage an der Tibia (20) dient, besteht, wobei die eine Platte (38) exakt senkrecht zur Hauptachse des Anschlagblockes (39) feststehend angeschlagen ist und für die Auflage frontal auf der Tibia (20) vorgesehen ist,
daß die femorale Sägelehre (12) im wesentlichen besteht aus einem Anlageblock (17), der exakt quaderförmig ausgebildet ist und mit seiner größten Stirnfläche zur frontalen Anlage an dem Femur (10) vorgesehen ist, derart, daß er um eine Schwenkachse (S) schwenkbar ist, und
daß das Tibiateil (35) und die femorale Sägelehre (12) dazu vorgesehen sind, in situ so zueinander in Lagebeziehung gebracht zu werden, daß beim Verfahren der bewegbaren Platte (24) fort von der feststehenden Platte (38) zwischen dieser und der tibiawärtsweisenden Stirnfläche der Sägelehre (12) gegebenenfalls unter Ausführung einer Schwenkbewegung um die Schwenkachse (S) zumindest ein linienförmiger Kontakt entsteht.

2. Vorrichtung nach Anspruch 1, dadurch gekennzeichnet, daß der Anschlagblock (39) eine Reihe von Durchbohrungen (31) zur Aufnahme von Fixationsnägeln (30) aufweist.

3. Vorrichtung nach Anspruch 1 oder 2, dadurch gekennzeichnet, daß die bewegliche Platte (24) distal mit zwei Tibiaanschlägen (25) versehen ist.

4. Vorrichtung nach einem der Ansprüche 1 bis 3, dadurch gekennzeichnet, daß die bewegliche Platte (24) einen Führungskasten (26) trägt, in den zeitweilig ein Führungselement (27) einsetzbar ist, welches von ventral nach dorsal verläuft und an seinem dorsalen Ende eine Führungsbuchse (28) für einen Führungsspieß (29) aufweist, der in den Markraum der Tibia (20) einsetzbar ist.

5. Vorrichtung nach einem der Ansprüche 1 bis 4, dadurch gekennzeichnet, daß außen am Anschlagsblock (39) des Tibiateils (35) eine externe Ausrichthilfe (22) für das Tibiateil (35) angebracht ist, und zwar in Form eines Peilstabes.

6. Vorrichtung nach einem der Ansprüche 1 bis 5, dadurch gekennzeichnet, daß die Schwenkachse (S) für die femorale Sägelehre (12) gebildet ist durch einen femoralen Führungsspieß (11, 11a), welcher in den Markraum des Femurs (10) einsetzbar ist.

7. Vorrichtung nach einem der Ansprüche 1 bis 6, dadurch gekennzeichnet, daß die Schwenkachse (S) für die femorale Sägelehre (12) auf der Achse einer auf dem Anlageblock (17) ausgebildeten Exzenterbuchse (16) liegt.

8. Vorrichtung nach einem der Ansprüche 1 bis 7, dadurch gekennzeichnet, daß in dem Anlageblock (17) wenigstens ein Schlitz (18) vorgesehen ist, durch welchen ein Blatt einer oszillierenden Knochensäge greifen kann.

9. Vorrichtung nach einem der Ansprüche 1 bis 8, dadurch gekennzeichnet, daß vom Anlageblock (17) der Sägelehre (12) ein Winkelansatz (13) absteht, der einen femurwärts in einem Winkel von 90° zur ventral weisenden Stirnfläche des Anlageblockes (17) stehenden Schenkel (14) trägt, der an seinem distalen Ende einen Femurkontaktfühler (15) in Form eines senkrecht auf dem Schenkel (14) stehenden und zum Femur weisenden Bolzens (15) hält.

10. Vorrichtung nach einem der Ansprüche 1 bis 9, dadurch gekennzeichnet, daß auch die Sägelehre (12) an der frontalen Femurseite lösbar arretierbar ist mittels durch sie hindurchlaufender Durchbohrungen für die Aufnahme von Fixationsnägeln.

## Claims

1. Device for fixing resection surfaces on the femur and on the tibia in preparation for implantation of a total knee joint endoprosthesis, comprising essentially a tibia part (35) which can be locked releasably in the region of the tubercle of the tibia and precisely positioned there in reproducible manner and a femoral saw gauge (12), characterised in that the tibia part (35) essentially comprises two parallel plates (24, 38), which can be moved exactly parallel to one another by means of a suitable advancing device (23, 34), and a stop block (39) which serves for contact against the tibia (20), wherein one plate (38) is stopped firmly exactly vertically to the main axis of the stop block (39) and is provided for frontal contact on the tibia (20), in that the femoral saw gauge (12) essentially comprises a stop block (17) which is designed to be exactly cuboid, and its largest end-face is provided for frontal contact against the femur (10), such that it can be pivoted about a pivoting axis (S), and in that the tibia part (35) and the femoral saw gauge (12) are provided to be brought into positional relationship with one another in situ, so that then moving the movable plate (24) away from the fixed plate (38), at least linear contact is produced between the latter and the end-face of the saw gauge (12) pointing in the tibia direction, optionally while executing a pivoting movement about the pivoting axis (S).

2. Device according to claim 1, characterised in that the stop block (39) has a series of bores (31) for receiving fixing nails (30).

3. Device according to claim 1 or 2, characterised in that the movable plate (24) is provided distally with two tibia stops (25).

4. Device according to one of claims 1 to 3, characterised in that the movable plate (24) supports a guide cage (26), in which a guide element (27) can be inserted temporarily, which guide element (27) runs ventrally to dorsally and has at its dorsal end a guide bush (28) for a pointed guide bar (29) which can be inserted in the medullary space of the tibia (20).

5. Device according to one of claims 1 to 4, characterised in that an external alignment aid (22) for the tibia part (35) is attached externally to the stop block (39) of the tibia part (35), and specifically in the form of a gauge rod.

6. Device according to one of claims 1 to 5, characterised in that the pivoting axis (S) for the femoral saw gauge (12) is formed by a femoral pointed guide bar (11, 11a) which can be inserted in the medullary space of the femur (10).

7. Device according to one of claims 1 to 6, characterised in that the pivoting axis (S) for the femoral saw gauge (12) lies on the axis of an eccentric bushing (16) formed on the stop block (17).

8. Device according to one of claims 1 to 7, characterised in that at least one slot (18), through which a blade of an oscillating bone saw may pass, is provided in the stop block (17).

9. Device according to one of claims 1 to 8, characterised in that an angular attachment (13) projects from the stop block (17) of the saw gauge (12), and has an arm (14) in the femoral direction at an angle of 90° to the ventrally pointing end-face of the stop block (17), which arm (14) has at its distal end a femoral contact sensor (15) in the form of a bolt (15) standing vertically on the arm (14) and pointing towards the femur.

10. Device according to one of claims 1 to 9, characterised in that the saw gauge (12) can also be releasably locked on the frontal femur side by means of bores passing through it for receiving fixing nails.

## Revendications

1. Dispositif pour déterminer des surfaces de résection sur le fémur et le tibia, afin de préparer une implantation d'une endoprothèse totale de l'articulation du genou, formé essentiellement par une pièce tibiale (35), qui peut être bloquée de manière amovible dans la zone de la tubérosité du tibia dans une position reproductible avec précision, et par un calibre de sciage fémoral (12), caractérisé en ce que la pièce tibiale (35) est formée par deux plaques (24, 38) parallèles, qui peuvent être déplacées l'une vers l'autre dans une position exactement parallèle à l'aide d'un dispositif d'entraînement (23, 34) approprié, et par un bloc de butée (39) qui vient en appui contre le tibia (20), l'une des plaques étant montée de manière fixe, exactement perpendiculaire à l'axe principal du bloc de butée (39) et est prévue pour entrer en contact frontal avec le tibia (20),
en ce que le calibre de sciage fémoral (12) est formé essentiellement par un bloc de contact (17), qui est conçu exactement en forme de parallélépipède, et est prévu pour entrer en contact frontal par sa plus grande surface frontale avec le fémur (10), de manière à pouvoir pivoter autour d'un axe de rotation (S),
et en ce que la pièce tibiale (35) et le calibre de sciage fémoral (12) sont prévus pour être positionnés l'un par rapport l'autre in situ, de telle sorte que, lors du déplacement de la plaque mobile (24) pour l'éloigner de la plaque fixe (38), il s'établit au moins un contact linéaire entre ladite plaque mobile et la surface frontale, orientée vers le tibia, du calibre de sciage (12), le cas échéant, par un pivotement autour de l'axe de rotation (S).

2. Dispositif selon la revendication 1, caractérisé en ce que le bloc de butée (39) est muni d'une série de perçages (31), dans lesquels s'engagent des clous de fixation (30).

3. Dispositif selon la revendication 1 ou 2, caractérisé en ce que la plaque mobile (24) est munie de deux butées pour tibia (25) éloignées dans le sens distal.

4. Dispositif selon l'une quelconque des revendications 1 à 3, caractérisé en ce que la plaque mobile (24) est munie d'une boîte de guidage (26), dans laquelle il est possible d'introduire temporairement un élément de guidage (27), qui s'étend de la zone ventrale vers la zone dorsale et dont l'extrémité dorsale est munie d'une douille de guidage (28) pour une pointe de guidage (29), qui est poussée dans la zone de moelle du tibia (20).

5. Dispositif selon l'une quelconque des revendications 1 à 4, caractérisé en ce qu'un guide d'alignement externe (22) pour la pièce tibiale (35) est monté à l'extérieur contre le bloc de butée (39) de la pièce tibiale (35), et ledit guide d'alignement est conçu en forme de tige de repérage.

6. Dispositif selon l'une quelconque des revendications 1 à 5, caractérisé en ce que l'axe de rotation (S) du calibre de sciage fémoral (12) est représenté par une pointe de guidage (11, 11a), qui est poussée dans la zone de moelle du fémur (10).

7. Dispositif selon l'une quelconque des revendications 1 à 6, caractérisé en ce que l'axe de rotation (S) du calibre de sciage fémoral (12) est monté sur l'axe d'une douille d'excentrique (16) réalisée sur le bloc de contact (17).

8. Dispositif selon l'une quelconque des revendications 1 à 7, caractérisé en ce qu'il est prévu de réaliser au moins une fente (18) dans le bloc de contact (17), dans laquelle peut s'engager la lame d'une scie oscillante pour chirurgie osseuse.

9. Dispositif selon l'une quelconque des revendications 1 à 8, caractérisé en ce qu'une pièce en équerre (13) forme une saillie sur le bloc de contact (17) du calibre de sciage (12), laquelle pièce est munie d'une branche (14) orientée vers le fémur en formant exactement un angle de 90° avec la surface frontale, orientée dans le sens ventral, du bloc de contact (17), l'extrémité distale de ladite branche est munie d'un capteur de contact fémoral (15), en forme de goupille (15) orientée vers le fémur et exactement perpendiculaire à la branche (14).

10. Dispositif selon l'une quelconque des revendications 1 à 9, caractérisé en ce que le calibre de sciage (12) peut également être bloqué de manière amovible sur la face frontale du fémur, à l'aide de perçages réalisés dans ledit calibre et destinés à recevoir des clous de fixation.
